Europäisches Patentamt

European Patent Office

Office européen des brevets

(11) Publication number: **0 147 974 B1**

# EUROPEAN PATENT SPECIFICATION

(45) Date of publication of patent specification: **30.10.91**  (51) Int. Cl.5: **C12P  13/22**

(21) Application number: **84308611.7**

(22) Date of filing: **11.12.84**

The file contains technical information submitted after the application was filed and not included in this specification

(54) **Process for refining phenylalanine.**

(30) Priority: **26.12.83 JP 251035/83**

(43) Date of publication of application:
**10.07.85 Bulletin  85/28**

(45) Publication of the grant of the patent:
**30.10.91 Bulletin  91/44**

(84) Designated Contracting States:
**FR IT NL**

(56) References cited:

**CHEMICAL ABSTRACTS, vol. 99, no. 4, 25th July 1983, page 372, abstract no. 28491d, Columbus, Ohio, US; A. SANNOMIYA et al.: "Adsorption of amino acids on MR-resin adsorbents", & KAGAKU KOGAKU RONBUNSHU 1983, 9(2), 217-20**

**CHEMICAL ABSTRACTS, vol. 97, no. 5, 2nd August 1982, pages 620-621, abstract no. 39376e, Columbus, Ohio, US; D.J. PIETRZYK et al.: "Preparative liquid chromatographic separation of amino acids and peptides on Amberlite XAD-4", & J. LIQ. CHROMATOGR. 1982, 5(3), 443-61**

(73) Proprietor: **AJINOMOTO CO., INC.**
**5-8, Kyobashi 1-chome, Chuo-ku**
**Tokyo 104(JP)**

(72) Inventor: **Otani, Masaru**
**No.2-89, Shinmei-cho, Saiwai-ku**
**Kawasaki-shi Kanagawa-ken(JP)**
Inventor: **Sano, Chiaki**
**No 2-24-10, Tamagawa, Ota-ku**
**Tokyo(JP)**
Inventor: **Kusumoto, Isao**
**No. 4-24, Onimaru-cho, Saga-shi**
**Saga-ken(JP)**

(74) Representative: **Bond, Bentley George et al**
**Haseltine Lake & Co. 28 Southampton Buildings Chancery Lane**
**London WC2A 1AT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 81, no. 21, 25th November 1974, page 459, abstract no. 136527g, Columbus, Ohio, US; & SU-A-437 750 (INSTITUTE OF HETEROORGANIC COMPOUNDS ACADEMY OF SCIENCES, U.S.S.R) 30-07-1974

CHEMICAL ABSTRACTS, vol. 97, no. 22, 29th November 1982, page 374, abstract no. 188140a, Columbus, Ohio, US; M. SPECHT et al.: "Separation of phenylalanine and tyrosine from protein hydrolyzates", & PROC. INT. SYMP. CYCLODEXTRINS, 1st 1981 (Pub. 1982), 497-500

## Description

This invention relates to a new process for refining phenylalanine In particular it relates to a process for eliminating impurities such as tyrosine as the main impurity, amino acids, organic acids, and salts, from phenylalanine solutions.

Examples of such phenylalanine solutions are as follows: L-phenylalanine fermentation liquids containing L-tyrosine; intermediate process liquids used to recover L-phenylalanine from such fermentation liquids; dissolved liquids of the crude crystals of L-phenylalanine containing L-tyrosine obtained from such intermediate process liquids; and other phenylalanine solutions containing at least tyrosine as impurity and possibly containing other amino acids, salts, etc. The process of the present invention may be applied to any L-phenylalanine solution.

The present invention is particularly applicable to L-phenylalanine fermentation liquid, from which various by-products such as amino acids, etc., can be easily separated from the L-phenylalanine by recrystallization, etc., but from which L-tyrosine is hardly separable from the L-phenylalanine by conventional refining processes such as recrystallization processes and ion-exchange processes (see Japanese Patent Publication No. 3789/1958).

As the result of intensive and extensive studies, the present inventors have found it possible to refine phenylalanine by an extremely simple process.

According to the invention, there is provided a process for refining phenylalanine,which comprises bringing a phenylalanine solution containing tyrosine as an impurity and optionally one or more other impurities into contact with a particular nonporous synthetic adsorbent, namely SP-207, which is a styrene-divinylbenzene copolymer made by Mitsubishi Chemical Industries Ltd., whereby the phenylanine is selectively adsorbed on the adsorbent,and eluting and recovering the adsorbed phenylalanine.

In an embodiment of the invention, the process of separating phenylalanine from a phenylalanine solution containing tyrosine as the main impurity needs only contact of the solution with the adsorbent. Thus, the present invention is concerned with a process for obtaining a highly pure phenylalanine solution by contacting a phenylalanine solution containing tyrosine, etc., with the adsorbent, adsorbing only the phenylalanine on the resin, and then eluting the phenylalanine adsorbed on the resin by an appropriate solvent. As occasion demands, by concentrating this phenylalanine solution, highly pure phenylalanine crystals can be obtained.

As for the manner of contact of the phenylalanine solution with the adsorbent, a batch process or a process effected in a column can be used. However, it is preferred to carry out this invention as a chromatography process of that column type. For instance, by filling a column with the adsorbent and then passing the phenylalanine solution containing impurities such as tyrosine downwardly through the column, the adsorbent adsorbs phenylalanine selectively, while the other materials are eliminated as effluent.

The concentration of the phenylalanine solution is not critical, and it would be agreeable if only within the saturated solubility at the operational temperature of adsorption. The pH of the phenylalanine solution, considering that the quantity of adsorption reaches a maximum at the isoelectric point, can be maintained between about 3 and 9, preferably about 5 and 6. Moreover the upper limit of the operational temperature of adsorption will depend upon the heat-resistance of the adsorbent, because phenylalanine is stable even at high temperatures. The operational temperature of adsorption,according to the heat-resistance of the adsorbent, for instance, may be kept below 60 $^\circ$ C or within the range of 0 to 90 $^\circ$ C. Even within this temperature range, as the quantity of adsorption is varied in such a way that, for example, the quantity of phenylalanine adsorbed on the adsorbent may reach a maximum at around 30 $^\circ$ C, the best adsorption operational temperature should be selected according to a preliminary experiment.

Furthermore, when the concentration of salts (e.g. inorganic salts such as $Na_2SO_4$, $NaCl$, $NH_4Cl$, etc) in a phenylalanine solution increases, the quantity of phenylalanine adsorbed on the adsorbent also increases, so the adsorption may favorably be operated in accordance with a column system after these salts have been added to the aqueous phenylalanine solution (see Example 2).

Since the adsorbent has stronger affinity for phenylalanine than for tyrosine, if, irrespective of the quantity of tyrosine as the main impurity, the adsorption is operated in order to attain a saturated adsorption of phenylalanine, the phenylalanine recovered by the elution will not contain tyrosine. The saturation rate of the adsorption, relative to phenylalanine, depends upon the desired purity of the phenylalanine.

The elution for the recovery of the phenylalanine adsorbed on the adsorbent is not difficult. For instance, the elution of the phenylalanine can be easily performed by water, acid, alkali, lower aliphatic alcohol (e.g. methanol, ethanol, isopropyl alcohol, etc.), and mixtures thereof ( e.g. aqueous solutions of methanol, ethanol, isopropyl alcohol, etc.).

The invention will now be illustrated by the following Examples.

## Example 1

2.5 litres (pH = 6.0) of an aqueous solution containing 3 g/dl of L-phenylalanine and 40 mg/dl of L-tyrosine was passed through a 100 ml bed(80 cm in height and 4 cm in diameter) of non-polar highly porous synthetic adsorbent, namely SP-207, in the form of a column, at an SV (space velocity in elution) of 1, whereby L-phenylalanine was adsorbed selectively.

Then, the L-phenylalanine was eluted using water as an elution solvent(SV = 2). Consequently, the quantity of water required for the elution was 20 litres, and more than 98% of the L-phenylalanine adsorbed was eluted.

By the deposition of crystals by the concentration of the eluate, 65g of L-phenylalanine crystals not containing any L-tyrosine, etc., were obtained. The purity of these crystals is more than 99.5%; furthermore, no L-tyrosine was detected by T.L.C. (thin layer chromatography) analysis.

## Example 2

3.5 litres (pH = 5.0) of L-phenylalanine solution containing 3 g/dl of L-phenylalanine, 40 mg/dl of L-tyrosine, 100 mg/dl of each of L-glutamic acid, L-alanine and L-lysine, 10 g/dl of NaCl and 2 g/dl of Na$_2$SO$_4$ was passed through a 1000 ml bed (80 cm in height and 4 cm in diameter) of non-polar porous synthetic adsorbent, namely SP-207, in the form of a column, at an SV of 1, whereby L-phenylalaine was adsorbed selectively.

Then, the L-phenylalaine was eluted by an aqueous EtOH (1% v/v) and NaOH (0.2N) solution (SV = 2). The quantity of aqueous solution required for the elution was 3.5 litres, and more than 98% of the L-phenylalanine adsorbed was eluted.

By the desposition of crystals, by neutralization with HCl after concentration of the eluate, 80g of L-phenylalanine crystals not containing any L-tyrosine, etc, were obtained. The purity of these crystals was more than 99.5%; furthermore, no L-tyrosine or other amino acids were detected by T.L.C. analysis.

## Claims

1. A process for the selective adsorption of amino acids by bringing a solution of said amino acids into contact with a non-polar porous synthetic adsorbent, characterised in that said non-polar porous synthetic adsorbent is SP-207 and in that said process is a process for refining phenylalanine which comprises bringing a phenylalanine solution containing tyrosine as an impurity and optionally one or more other impurities into contact with said non-polar porous synthetic adsorbent whereby the phenylalanine is selectively adsorbed on the adsorbent, and eluting and recovering the adsorbed phenylalanine.

2. A process according to claim 1, wherein the adsorbent is in the form of a column, and wherein the phenylalanine solution is passed downwardly through the column.

3. A process according to claims 1 or 2 wherein the phenylalanine solution contains a salt.

## Revendications

1. Procédé d'adsorption sélective d'acides aminés par mise en contact d'une solution desdits acides aminés avec un adsorbant synthétique poreux non polaire, caractérisé en ce que ledit adsorbant synthétique poreux non polaire est du SP-207 et en ce que ledit procédé est un procédé d'épuration de la phénylalanine comprenant la mise en contact d'une solution de phénylalanine contenant de la tyrosine comme impureté et éventuellement une ou plusieurs autres impuretés avec ledit adsorbant synthétique poreux non polaire, la phénylalanine étant adsorbée sélectivement sur l'adsorbant, et l'élution et la récupération de la phénylalanine adsorbée.

2. Procédé selon la revendication 1, dans lequel l'adsorbant a la forme d'une colonne et dans lequel la solution de phénylalanine est passée de haut en bas sur la colonne.

3. Procédé selon la revendication 1 ou 2, dans lequel la solution de phénylalanine contient un sel.

## Patentansprüche

1. Verfahren zur selektiven Adsorption von Aminosäuren indem eine Lösung dieser Aminosäuren mit einem unpolaren, porösen, synthetischen Adsorbens in Kontakt gebracht wird, dadurch gekennzeichnet, daß das unpolare, poröse, synthetische Adsorbens SP-207 ist, und daß das Verfahren ein Verfahren zur Reinigung von Phenylalanin ist, welches das Inkontaktbringen einer Phenylalaninlösung, die Tyrosin als Verunreinigung und, gegebenenfalls, eine oder mehrere andere Verunreinigungen enthält, mit dem unpolaren, porösen, synthetischen Adsorbens, wodurch das Phenylalanin selektiv von dem Adsorbens adsorbiert wird, und das Eluieren und die Gewinnung des adsorbierten Phenylalanins, umfaßt.

2. Verfahren nach Anspruch 1, wobei das Adsorbens in der Form einer Säule vorliegt, und wobei die Phenylalaninlösung in Richtung nach unten über die Säule geleitet wird.

3. Verfahren nach Anspruch 1 oder 2, wobei die Phenylalaninlösung ein Salz enthält.